# EUROPÄISCHE PATENTSCHRIFT

(11) **EP 0 593 633 B1**
(45) Veröffentlichungstag und Bekanntmachung des Hinweises auf die Patenterteilung: **16.12.1998**
(21) Anmeldenummer: 92915328.6
(22) Anmeldetag: 09.07.1992
(51) Int. Cl.: A61K 31/72, C08B 35/02, C08B 31/04, C08L 3/16

(54) **METABOLISIERBARER PLASMAERSATZ**
METABOLIZABLE PLASMA SUBSTITUTE
PLASMA ARTIFICIEL METABOLISABLE

(30) Priorität: 11.07.1991 DE 4122999
(43) Veröffentlichungstag der Anmeldung: 27.04.1994
(73) Patentinhaber: FRIKA Pharmazeutische Fabrik Gesellschaft m.b.H., 1030 Wien (AT)
(72) Erfinder: FÖRSTER, Harald, D-6000 Frankfurt/Main 71 (DE); ASSKALI, Fatima, D-6000 Frankfurt/Main 71 (DE); NITSCH, Ernst, A-4040 Linz (AT)
(74) Vertreter: Huber, Bernhard, Dipl.-Chem.
(86) Internationale Anmeldenummer: EP9201552
(87) Internationale Veröffentlichungsnummer: WO9300914

(56) Entgegenhaltungen:
- WO-A-93/00939
- WO-A-93/01217
- DE-A- 2 736 036
- DE-A- 3 313 600

## Beschreibung

Die Erfindung betrifft Plasmaersatzlösungen, die als kolloidalen Bestandteil einen Stärkeester enthalten.

Bei zahlreichen Indikationen, z.B. großem Blutverlust, ist es erforderlich, kolloidosmotisch wirksame Substanzen als Plasmaersatz (Plasmastreckmittel) zu verwenden. Diese Substanzen dienen dazu, den kolloidosmotischen Druck innerhalb des Intravasalraumes aufrechtzuerhalten sowie zur Blutverdünnung (Hämodilution), z.B. bei der Behandlung von Durchblutungsstörungen. Für derartige Plasmaersatzmittel und Hämodilutionsmittel besteht ein ständig steigender Bedarf.

Es ist bekannt, für solche Blutplasmaersatzmittel Polyvinylpyrrolidon, Gelatinederivate, Dextran und Hydroxyethylstärke zu verwenden (vgl. z.B. US-A 3,937,821; DE-A 33 13 600; Römpp Chemielexikon, 9. Auflage, Seite 919, 1509).

Zur Zeit werden zur klinischen Verwendung vorwiegend die Polysaccharide Dextran und Hydroxyethylstärke eingesetzt. Der wesentliche Nachteil von Dextran ist, daß aufgrund des Vorhandenseins von preformierten Antikörpern anaphylaktische Reaktionen auftreten können, die die Anwendung dieser Substanz gefährlich machen oder sie durch eine zwingende Vorbehandlung mit einem neutralisierenden niedermolekularen Hapten komplizieren. Bei einer kurzzeitigen, unmittelbaren Anwendung von Hydroxyethylstärke wurden zwar nur selten unmittelbare Nebenwirkungen beobachtet; die längerdauernde Anwendung von Hydroxyethylstärke wird jedoch durch deren Speicherung, insbesondere im retikuloendothelialen Gewebe, stark eingeschränkt. Die Hydroxyethylstärke kann durch körpereigene Enzyme nur langsam bzw. unvollständig abgebaut werden, weil die Veretherung den Angriff körpereigener Glycosidasen erschwert. Es ist zwar bisher noch nicht mit Sicherheit bekannt, ob diese Speicherung die Funktionsfähigkeit des retikuloendothelialen Systems beeinträchtigt, doch wird z.B. das Auftreten von Juckreiz als Nebenwirkung, die bei der Anwendung von Hydroxyethylstärke zu beobachten ist, mit dieser Speicherung in Zusammenhang gebracht.

Aufgabe der vorliegenden Erfindung ist es deshalb, Plasmaersatzmittel bereitzustellen, die die vorstehend genannten Nachteile der bisher üblichen Plasmaersatzmittel vermeiden und bei deren Anwendung keine wesentlichen Nebenwirkungen zu erwarten sind. Insbesondere soll die Grundlage für die Plasmaersatzmittel eine Substanz sein, gegen die keine preformierten Antikörper vorhanden sind und die im Organismus nicht in stärkerem Ausmaß gespeichert wird. Diese Aufgabe wird mit der vorliegenden Erfindung gelöst.

Gegenstand der Erfindung gemäß Anspruch 1 ist daher die Verwendung eines Stärkeesters, bei welchem die Stärke mit Acylgruppen von Monocarbonsäuren oder Dicarbonsäuren oder gemischen aus Mono- und Dicarbonsäuren mit jeweils 2 bis 6 C-Atomen substituiert ist, mit einem Molekulargewicht (Mw) > 20000 Dalton und einer molaren Substitution von 0,1 bis 1,5, zur Herstellung eines Blutplasmaersatzmittels.

Zweckmäßige Ausgestaltungen davon sind Gegenstand der Ansprüche 2 bis 8.

Es wurde gefunden, daß sich Stärkeester mit organischen Säuren, wie z.B. Acetylstärke, sehr gut für die Verwendung in Plasmaersatzmittel eignen. Stärkeester werden durch Esterasen gespalten. Die dabei entstehende Stärke ist mit dem körpereigenen Glykogen so nahe verwandt, daß das Entstehen von Antikörpern auszuschließen ist. Sie wird wie körpereigenes Glykogen durch Glykosidasen in niedermolekulare Bruchstücke (Oligosaccharide, Isomaltose, Maltose) gespalten, und kann bis zu Glucose abgebaut werden. Die beim Abbau von Stärkeestern entstehenden organischen Säuren (wie z.B. Essigsäure) werden entweder metabolisiert oder über die Niere ausgeschieden. Die beim Abbau der Stärke entstehende Glucose unterliegt dem physiologischen Stoffwechsel.

Bei den erfindungsgemäß als kolloidaler Bestandteil von Plasmaersatzlösungen verwendeten Stärkeestern handelt es sich also um Hydrokolloide, die einer physiologischen (körpereigenen) Substanz sehr ähnlich sind, und die durch enzymatischen Abbau weitgehend vollständig metabolisiert werden können. Sie führen nach den bisher vorliegenden Ergebnissen nicht zur Antikörperbildung und eine Speicherung der erfindungsgemäß verwendeten Säureester in größerem Umfang oder über längere Zeiträume in den verschiedenen Organen ist auszuschließen.

Die Untersuchung der Organe von mit Acetylstärke behandelten Tieren ergab, daß nur unmittelbar nach der Applikation von Acetylstärke und 3 Stunden nach der Applikation eine Ablagerung von Acetylstärke in geringer Menge festgestellt wurde, insbesondere in den Nieren und in den Lungen. Bei diesen Organen handelt es sich um stark durchblutete Organe und es kann deshalb nicht ausgeschlossen werden, daß die im Blut vorhandene Acetylstärke bei der Analyse miterfaßt wurde. Es wurde jedoch festgestellt, daß insbesondere die Milz als Vertreter des retikuloendothelialen Systems kaum eine Ablagerung von Acetylstärke zeigte. Insbesondere war mehrere Tage nach Applikation von Acetylstärke in einer einmaligen Dosierung von 2 bis 8 g/kg Körpergewicht bei Ratten keine gespeicherte Acetylstärke mehr nachweisbar. Dies stellt einen wesentlichen Vorteil dar gegenüber in Plasmaersatzmitteln bisher gebräuchlichen Hydrokolloiden (z.B. Dextran oder Hydroxyethylstärke); nach Applikation von Dextran oder von Hydroxyethylstärke in vergleichbaren Mengen bei Ratten waren unter gleichen Bedingungen auch Wochen und Monate später noch Ablagerungen von Dextran oder von Hydroxyethylstärke in der Leber, der Milz, den Lungen und den Nieren festzustellen.

In ihrer Wirkung als Plasmaersatzmittel (Plasmastreckmittel) sind die erfindungsgemäßen Stärkeester mit den bisher üblichen für derartige Mittel verwendeten Polysacchariden, insbesondere Hydroxyethylstärke und Dextran, vergleichbar, besitzen diesen gegenüber aber die vorstehend genannten Vorteile. Sie eignen sich deshalb hervorragend als kolloidaler Bestandteil von Plasmaersatzlösungen (Plasmastreckmitteln).

Die erfindungsgemäß eingesetzten Stärkeester sind Stärkeester mit einer molaren Substitution MS von 0,1 bis 1,5. Stärkeester sind Ester mit organischen Carbonsäuren und insbesondere mit aliphatischen Mono- und Dicarbonsäuren mit 2 bis 6 Kohlenstoffatomen, wie z.B. Essigsäure, Propionsäure, Buttersäure, Isobuttersäure, Bernsteinsäure und Maleinsäure. Die molare Substitution MS beträgt vorzugsweise 0,3 bis 0,5. Ein erfindungsgemäß besonders bevorzugter Stärkeester ist Acetylstärke, insbesondere solche mit einem Molekulargewicht (Mw) von 100000 bis 200000 Dalton und einer molaren Substitution MS von 0,3 bis 0,5.

Die Stärkeester leiten sich z.B. von wachsartiger Milo(Sorghum)-Stärke, wachsartiger Maisstärke oder wachsartiger Reisstärke ab. In erster Linie werden aus nativen Produkten durch partielle Hydrolyse erhaltene Fraktionen mit zweckentsprechendem Molekulargewicht eingesetzt, und insbesondere durch partiellen hydrolytischen Abbau von an Amylopektin reicher Stärke und nachfolgender partieller Veresterung erhaltene Stärkeester.

Die geeignetste molare Substitution ist insbesondere auch von der Art des verwendeten Stärkeesters (Art der Ausgangsstärke, Molekulargewicht und/oder Estergruppierung) abhängig. Er beeinflußt auch die Verweildauer im Plasma und damit die Wirkdauer der eingesetzten Stärkeester, die sich deshalb durch Modifikation des Substitutionsgrades steuern läßt.

Die erfindungsgemäß verwendeten Stärkeester besitzen eine Untergrenze des mittleren Molekulargewichts (Mw) von ca. 20000 Dalton. Diese untere Grenze ist dadurch gegeben, daß das Molekulargewicht der Stärkeester oberhalb der Nierenschwelle liegen soll.

Vorzugsweise werden Stärkeester mit einem mittleren Molekulargewicht (Mw) von 40000 bis 1000000 Dalton und vorzugsweise von 100000 bis 450000 Dalton eingesetzt.

Zur Anwendung als Plasmaersatz oder zur Hämodilution ist neben dem Stärkeester als kolloidaler Bestandteil der Zusatz von osmotisch wirksamen Substanzen erforderlich. Das erfindungsgemäße Plasmaersatzmittel enthält den Stärkeester deshalb in Kombination mit einem physiologisch annehmbaren, osmotisch wirksamen Elektrolyten und/oder anderen osmotisch wirksamen Substanzen in wäßriger Lösung. Die Konzentration an Stärkeester beträgt vorzugsweise 1 bis 12 % Gew/Vol, insbesondere 3 bis 10 % Gew/Vol, bezogen auf das gesamte Plasmaersatzmittel.

Als physiologisch annehmbare Elektrolyte kommen solche Elektrolyte und deren Gemische in Frage, wie sie üblicherweise in derartigen Plasmaersatzmitteln verwendet werden, also insbesondere z.B. Natriumchlorid, Calciumchlorid, Salze der niederen Carbonsäuren mit 2 bis 4 Kohlenstoffatomen, insbesondere Essigsäure.

Die andere osmotisch wirksame Substanz kann eine niedermolekulare organische Verbindung sein und besteht vorzugsweise aus mehrwertigen Alkoholen, Monosacchariden, Disacchariden, wie z.B. Glycerin, Sorbit, Maltose und in erster Linie Glucose und/oder Aminosäuren.

In der Regel werden als osmotisch wirksame Elektrolyte und andere osmotisch wirksame Substanzen solche eingesetzt, die eine Osmolarität von 200 bis 450 mosmol/kg ergeben und vorzugsweise von 250 bis 350 mosmol/kg.

Die erfindungsgemäßen Plasmaersatzmittel können einen oder mehrere erfindungsgemäße Stärkeester in Kombination mit einer oder mehreren der osmotisch wirksamen Substanzen (Elektrolyt und/oder andere osmotisch wirksamen Substanzen) in Wasser enthalten.

Die erfindungsgemäß eingesetzten Stärkeester können nach dem in der gleichzeitig unter der Bezeichnung "Verfahren zur Herstellung von Stärkeestern für klinische, insbesondere parenterale Anwendung" der gleichen Anmelderin, Aktenzeichen P 41 23 000.0 hergestellt werden. Die Herstellung der Plasmaersatzmittel erfolgt auf an sich bekannte Weise, z.B. durch Einmischen der Komponenten in Wasser oder eine wäßrige Elektrolytlösung, und die Stärkeester werden vorzugsweise in Form von durch Trocknen, z.B. durch Sprühtrocknung, Trommeltrocknung oder Vakuumtrocknung, und Mahlen erhaltene Pulver eingesetzt.

Die nachfolgenden Beispiele sollen die Erfindung näher erläutern.

### Beispiel 1

Herstellung von Plasmaersatzlösungen

Als Stärkeester wurde Acetylstärke mit einem mittleren Molekulargewicht (Mw) von ca. 200000 Dalton und mit einem Substitutionsgrad von 0,3 oder 0,5 eingesetzt.

Damit wurden die folgenden Plasmaersatzlösungen hergestellt:
1. Acetylstärkelösung in einer Konzentration von 3, 6 und 10 Gew.-% in physiologischer Kochsalzlösung (0,9 Gew.-%)
2. Acetylstärkelösung in einer Konzentration von 6 Gew.-% in 2,5 Gew.-%iger Glycerinlösung (elektrolytfreie Plasmaersatzlösung)

### Beispiel 2

Untersuchungen zur biologischen Abbaubarkeit

Acetylstärkelösungen gemäß Beispiel 1 mit einer Konzentration von 3, 6 und 10 Gew.-% Acetylstärke (Substitutionsgrad 0,3 oder 0,5) in physiologischer Kochsalzlösung wurden Ratten intravenös verabreicht (18 ml in 3 Stunden).

Die Infusionen wurden sehr gut toleriert, und es wurden keine unmittelbaren Nebenwirkungen festgestellt. Unmittelbar nach Beendigung der Infusionen waren in Abhängigkeit von der Dosis Blutspiegel von Acetylstärke vorhanden (6 bis 25 mg/ml). Diese Blutspiegel waren mit denen vergleichbar, die unter Verwendung von Hydroxyethylstärkelösungen (HES 200/0,5; Mw ca. 200000 Dalton, molare Substitution 0,5 Mol) erhalten wurden. Auch 3 Stunden nach Beendigung der Infusion war noch Acetylstärke im Blut der Tiere nachweisbar (1,0 bis 10 mg/ml); diese Mengen entsprachen ebenfalls den Mengen, die mit der Hydroxyethylstärkelösung (HES 200/0,5) unter vergleichbaren Bedingungen erhalten wurden. 24 Stunden nach Infusionsende war bei den behandelten Tieren jedoch keine Acetylstärke mehr im Blut nachweisbar.

Die Figur 1 zeigt den Serumgehalt (in mg/ml), der nach dreistündiger Infusion sofort nach Infusionsende (A bis E) bzw. 3 Stunden nach Infusionsende (F, G, H) festgestellt wurde.

Die Figur 2 zeigt den Gehalt an Hydrokolloid in der Niere (in mg/g), der sofort nach Infusionsende (B, C, D, E und NaCl), 3 Stunden nach Infusionsende (F, G) und 18 bis 24 Stunden nach Infusionsende (I, J, K, L) festgestellt wurde.

Die Figur 3 zeigt die entsprechenden Angaben gemäß Figur 2 für die Lunge.

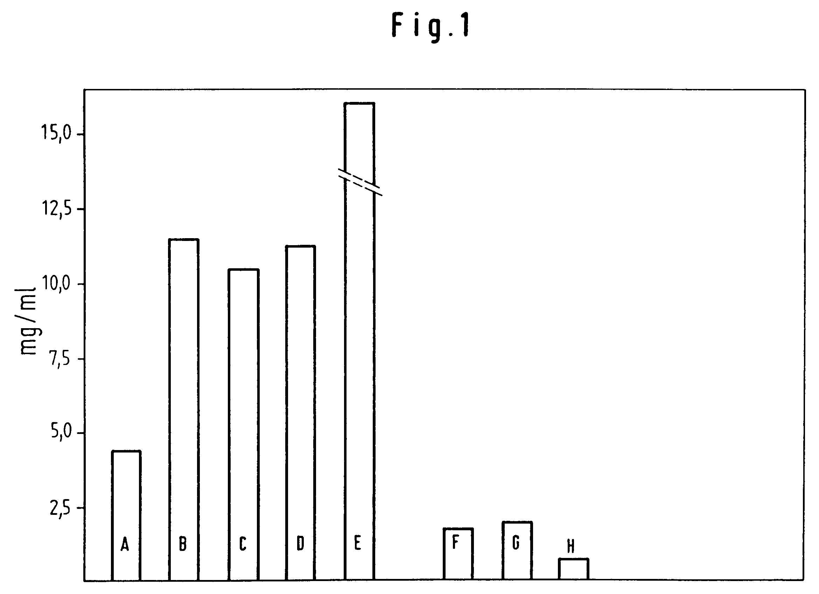

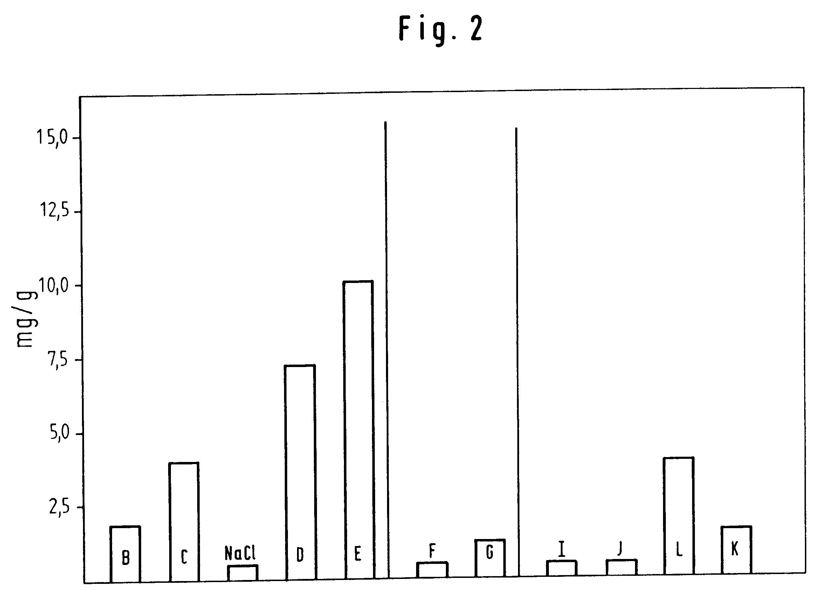

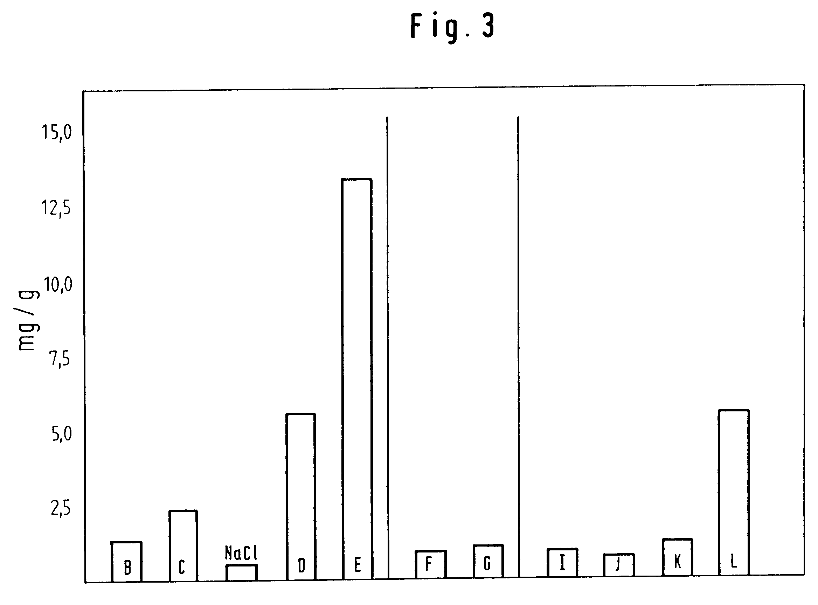

Die Angaben A bis L in den Figuren 1 bis 3 beziehen sich auf die folgenden Plasmaersatzlösungen:

A - 3 % Acetylstärke; B - 6 % Acetylstärke; C - 10 % Acetylstärke; D - 6 % HES 200/0,5; E - 10 % HES 200/0,5; F - 6 % Acetylstärke; G - 10 % Acetylstärke; H - 3 % Acetylstärke; I - 6 % Acetylstärke; J - 10 % Acetylstärke; K - 6 % HES 200/0,5; L - 10 % HES 200/0,5. NaCl = Natriumchlorid.

## Patentansprüche

1. Verwendung eines Stärkeesters, bei welchem die Stärke mit Acylgruppen von Monocarbonsäuren oder Dicarbonsäuren oder Gemischen aus Mono- und Dicarbonsäuren mit jeweils 2 bis 6 C-Atomen substituiert ist, wobei der Stärkeester ein Molekulargewicht (Mw) > 20000 Dalton und eine molare Substitution von 0,1 bis 1,5 aufweist, zur Herstellung eines Blutplasmaersatzmittels.

2. Verwendung nach Anspruch 1,
**dadurch gekennzeichnet,**
daß die molare Substitution 0,2 bis 0,7, insbesondere 0,3 bis 0,5 ist.

3. Verwendung nach Anspruch 1 oder 2,
**dadurch gekennzeichnet,**
daß das Molekulargewicht (Mw) 40000 bis 1000000 Dalton, insbesondere 100000 bis 450000 Dalton ist.

4. Verwendung nach einem der Ansprüche 1 bis 3,
**dadurch gekennzeichnet,**
daß der Stärkeester Acetylstärke ist.

5. Verwendung nach Anspruch 4,
**dadurch gekennzeichnet,**
daß die Acetylstärke eine Acetylstärke mit einem Molekulargewicht (Mw) von 100000 bis 200000 Dalton und einer molaren Substitution von 0,3 bis 0,5 ist.

6. Blutplasmaersatzmittel,
**dadurch gekennzeichnet,**
daß es 1 bis 12 Gew.-%, insbesondere 3 bis 10 Gew.-% eines Stärkeesters enthält, bei welchem die Stärke mit Acylgruppen von Monocarbonsäuren oder Dicarbonsäuren oder Gemischen aus Mono- und Dicarbonsäuren mit jeweils 2 bis 6 C-Atomen substituiert ist, wobei der Stärkeester ein Molekulargewicht (Mw) > 20000 Dalton und eine molare Substitution von 0,1 bis 1,5 aufweist, bezogen auf das Plasmaersatzmittel, in Kombination mit einem physiologisch annehmbaren osmotisch wirksamen Elektrolyten und/oder einer oder mehreren anderen osmotisch wirksamen Substanzen in wäßriger Lösung.

7. Mittel nach Anspruch 6,
**dadurch gekennzeichnet,**
daß die anderen osmotisch wirksamen Substanzen mehrwertige Alkohohle, Monosaccharide, Disaccharide und/oder Aminosäuren sind.

8. Mittel nach Anspruch 7,
**dadurch gekennzeichnet,**
daß die andere osmotisch wirksame Substanz Glycerin und/oder Glucose ist.

## Claims

1. Use of a starch ester in which the starch is substituted with acyl groups of monocarboxylic acids or dicarboxylic acids or mixtures of mono-and dicarboxylic acids each with 2 to 6 C atoms, the starch ester having a molecular weight (Mw) of > 20000 Daltons and a molar substitution of 0.1 to 1.5 for the production of a blood plasma substitute.

2. Use as claimed in claim 1,
**wherein**
the molar substitution is 0.2 to 0.7, in particular 0.3 to 0.5.

3. Use as claimed in claim 1 or 2,
**wherein**
the molecular weight (Mw) is 40000 to 1000000 Daltons, in particular 100000 to 450000 Daltons.

4. Use as claimed in one of the claims 1 to 3,
**wherein**
the starch ester is acetyl starch.

5. Use as claimed in claim 4,
**wherein**
the acetyl starch is an acetyl starch with a molecular weight (Mw) of 100000 to 200000 Daltons and a molar substitution of 0.3 to 0.5.

6. Blood plasma substitute
**wherein**
it contains 1 to 12 % by weight, in particular 3 to 10 % by weight starch ester relative to the plasma substitute in which the starch is substituted with acyl groups of monocarboxylic acids or dicarboxylic acids or mixtures of mono- and dicarboxylic acids each with 2 to 6 C atoms, the starch ester having a molecular weight (Mw) of > 20000 Daltons and a molar substitution of 0.1 to 1.5, in combination with a physiologically acceptable osmotically active electrolyte and/or one or several other osmotically active substances in aqueous solution.

7. Agent as claimed in claim 6,
**wherein**
the other osmotically active substances are polyvalent alcohols, monosaccharides, disaccharides and/or amino acids.

8. Agent as claimed in claim 7,
**wherein**
the other osmotically active substance is glycerol and/or glucose.

## Revendications

1. Utilisation d'un ester d'amidon dans lequel l'amidon est substitué par des groupes acyle des acides monocarboxyliques ou dicarboxyliques ou des mélanges à partir d'acides mono- et dicarboxyliques avec chaque fois 2 à 6 atomes C, l'ester d'amidon présentant un poids moléculaire (Mw) > 20 000 Dalton et une substitution molaire de 0,1 à 1,5 pour la préparation d'un plasma sanguin artificiel.

2. Utilisation selon la revendication 1,
caractérisée en ce que
la substitution molaire est de 0,2 à 0,7, en particulier de 0,3 à 0,5.

3. Utilisation selon la revendication 1 ou 2,
caractérisée en ce que
le poids moléculaire (Mw) est de 40 000 à 1 000 000 Dalton, en particulier 100 000 à 450 000 Dalton.

4. Utilisation selon l'une des revendications 1 à 3,
caractérisée en ce que
l'ester d'amidon est de l'amidon d'acétyle.

5. Utilisation selon la revendication 4,
caractérisée en ce que
l'amidon d'acétyle est un amidon d'acétyle ayant un poids moléculaire (Mw) de 100 000 à 200 000 Dalton et une substitution molaire de 0,3 à 0,5.

6. Plasma sanguin artificiel
caractérisé en ce qu'
il contient de 1 à 12 %, en particulier 3 à 10 % en poids d'un ester d'amidon dans lequel l'amidon est substitué par des groupes acyle des acides monocarboxyliques ou dicarboxyliques ou de mélanges à partir d'acides mono- et dicarboxyliques avec chaque fois 2 à 6 atomes C, l'ester d'amidon ayant un poids moléculaire (Mw) inférieur à 200 000 Dalton et une substitution molaire de 0,1 à 1,5, rapportée au plasma de substitution, en combinaison avec un électrolyte osmotiquement efficace acceptable physiologiquement et/ou une substance ou plusieurs autres substances osmotiquement efficaces dans une solution aqueuse.

7. Moyen selon la revendication 6,
caractérisé en ce que
les autres substances osmotiquement efficaces sont des alcools polyvalents, des monosaccharides, des disaccharides et/ou des acides aminés.

8. Moyen selon la revendication 7,
caractérisé en ce que
l'autre substance osmotiquement efficace est de la glycérine et/ou du glucose.
